# EUROPEAN PATENT SPECIFICATION

(11) **EP 1 227 735 B1**
(45) Date of publication and mention of the grant of the patent: **22.10.2003**
(21) Application number: 00941642.1
(22) Date of filing: 22.06.2000
(51) Int. Cl.: A23G 3/30, A61K 7/00

(54) **CHEWING GUM COMPOSITIONS**
KAUGUMMIZUSAMMENSETZUNG
COMPOSITIONS DE GOMME A MACHER

(30) Priority: 12.11.1999 US 165351 P; 04.02.2000 US 180352 P
(43) Date of publication of application: 07.08.2002
(73) Proprietor: The Procter & Gamble Company, Cincinnati, Ohio 45202 (US)
(72) Inventor: DAY, Trevor, Neil, Windsor SL4 3SG (GB); WHITE, Donald, James, Jr., Fairfield, OH 45014 (US)
(74) Representative: Clemo, Nicholas Graham
(86) International application number: US0017177
(87) International publication number: WO01039606

(56) References cited:
- EP-A- 0 309 414
- EP-A- 0 333 301
- EP-A- 0 414 932
- WO-A-95/07683
- WO-A-97/02011
- US-A- 3 932 603
- US-A- 4 765 984
- US-A- 4 867 989
- US-A- 5 380 530
- US-A- 5 407 661
- US-A- 5 783 172
- US-A- 5 833 954
- PATENT ABSTRACTS OF JAPAN vol. 1997, no. 03, 31 March 1997 (1997-03-31) & JP 08 301742 A (EZAKI GLICO CO LTD), 19 November 1996 (1996-11-19)

## Description

### BACKGROUND OF THE INVENTION

The present invention relates to chewing gum compositions containing polymeric particulate polyphosphates. These polyphosphates may provide novel surface conditioning reaction to oral surfaces such as the teeth and mucosa. This leads to improved cleaning impression. The present invention further relates to compositions wherein the chewing gum has a crunchy texture conferred to it by the polyphosphate particles.

Polymeric surface active agents, such as polyphosphates, are known in the oral care art. US 5,380,530 discloses chewing gums containing polydimethylsiloxanes. WO 97/02011 discloses chewing gums containing aminoalkylsilicones. Although disclosed in chewing gum compositions, these ingredients are most commonly found in dentifrice compositions. Polyphosphates are known to provide an anti-calculus benefit as stated in U.S. Patent 5.094,844, US 4808401 and EP 0333301 issued to Gaffar et al.. Polyphosphates are also known to provide a buffering effect within zinc comprising oral compositions as stated in U.S. Patent 4,170,632 and US 4,170,633 both to Wagenknecht, deceased et al. Other chewing gum patents disclosing a polyphosphate include U.S. patent 5,702,687 issued to Miskewitzand EP0,387,024 issued to McClanahan, and EP 309414 to Avantgarde S.p.A. US 4,765,984 and US 5,833,954 disclose chewable products containing phosphates.

Prior art also exists wherein particulate matter has been incorporated into chewing gum compositions such that the resultant product does have a somewhat "crunchy" texture. For example chewing gums with a "crunchy" exterior coating have been previously disclosed such as the hard sugar coated gums disclosed in US 4,486,511 and US 4,792,453 and gums with surface printed solid particles disclosed in US 3,962,463. Such disclosures are limited to a "crunchy" exterior surface of the gum and thus do not provide an overall texture sensation. Furthermore, confectionery gums wherein sugars and sugar substitutes (such as isomalt, candy and the like), sometimes in conjunction with freeze dried food stuffs, are distributed throughout the body of the gum for the purpose, at least in part, of providing a "crunchy" texture are also known (FR 2,748,902; GB 950,811; EP 017,691; US 5,958472, and US 5,017,385). Whilst these latter disclosures do provide useful advances in conferring a "crunchy" sensation to the gum they do so by the use of food stuffs and not by use of the oral care active itself.

Surprisingly, research has now revealed additional benefits of chewing gums containing polymeric surface active agents, particularly when the polymeric surface active agent is a polyphosphate, even more particularly a particulate polyphosphate. These new benefits are related to effects on the surface chemical characteristics of mucosal and tooth surfaces which provide remarkable cleaning impression and positive mouth feel characteristics for extended periods of time during and following use. These effects have now been shown to be correlated to effects on oral surface energy characteristics including modification of surface hydrophilic and hydrophobic properties. Additionally, when certain polyphosphates, particularly of a particulate nature, are incorporated within the gum they can confer a "crunchy" texture to the product which lasts throughout the initial minutes of mastication and which reinforces for the consumer the oral care benefit of the product. Furthermore, due to the soluble nature of the polyphosphate materials the "crunchy" texture disappears over time leaving no gritty residue.

The surface conditioning effects can measured using several different methods. The surface conditioning effects on a subject's teeth and oral mucosa can be measured in vivo. These measurements include consumer responses on questions concerning clean teeth and smooth teeth. Other in vivo measurement include the water contact angle on the tooth surface and on the mucosa surfaces. The surface conditioning effects can also be measured in considerable detail through in vitro methods. In vitro methods are made over time to measure surface free energies and pellicle film thickness and composition.

It has also been found that the polymeric surface active agent can help to reduce the astringency of a metallic ion. Additionally, this reduction in astringency can occur without significantly reducing the efficacy of the metallic ion and without significantly reducing the efficacy and the surface conditioning effects of the polymeric surface active agent.

It is an object of the present invention to provide chewing gum compositions containing polymeric surface active agents which provide improved intraoral cleaning impression and smooth tooth surface impression derived from the chemical control of tooth and mucosal surface energy characteristics.

It is a further object of this invention to provide a chewing gum which has a "crunchy" texture during the initial minutes of mastication disappearing over time to leave no gritty residue and wherein this "crunchy" texture is provided by a polyphosphate.

These and other objects of the present invention will become readily apparent from the detailed description which follows.

All percentages and ratios used herein are by weight of the specific chewing gum composition, unless otherwise specified. All measurements are made at 25° C, unless otherwise specified.

### SUMMARY OF THE INVENTION

This invention relates to a chewing gum composition which has a crunchy texture wherein the crunchy texture is conferred to the gum by the inclusion of particulate polyphosphate material within the composition. The "crunchy" texture can be attained when the polyphosphate material used has a particle size of between 100µm and 2000µm. Furthermore, by ensuring that the polyphosphate is at least sparingly soluble the "crunch" will slowly disappear to leave a non gritty gum.

### DETAILED DESCRIPTION OF THE INVENTION

The chewing gum compositions of the present invention may be in the form of a conventional chewing gum or any other product form which is suitable for chewing. Suitable physical forms include sticks, dragees, chiclets, and batons. The chewing gum may also be a digestible or dissolvable gum suitable for chewing. A chewing gum is typically retained in the oral cavity for a time sufficient to allow ingredients released to contact substantially all of the dental surfaces and/or oral tissues for purposes of oral activity.

The term "carrier materials" as used herein means any safe and effective additional chewing gum components used in the chewing gum compositions of the present invention. Such materials include abrasive polishing materials, elastomers, resins, plasticisers, fats, solvents, waxes, emulsifiers, softeners, bulking agents, sweeteners, absorbents, orally active metallic ions, cationic material, fluoride ion sources, additional anticalculus agents, antimicrobial agents, buffers, whitening agents, alkali metal bicarbonate salts, thickening materials, humectants, water, surfactants, titanium dioxide, flavoring agents, xylitol, coloring agents, and mixtures thereof.

The present compositions comprise essential components, as well as optional components. The essential and optional components of the compositions of the present invention are described in the following paragraphs.

### Polymeric Surface Active Agent

The present invention includes a polymeric surface active agent which is a polyphosphate. The agents will provide surface conditioning effects. These agents may also be tartar control or anticalculus agents and may also provide stain control and reduction in plaque. The polymeric surface active agents will also provide a clean teeth and longer lasting clean teeth and mouthfeel.

A polyphosphate is generally understood to consist of two or more phosphate molecules arranged primarily in a linear configuration, although some cyclic derivatives may be present. Although pyrophosphates and tripolyphosphate are a polyphosphates, the polyphosphates desired are those having around four or more phosphate molecules so that one or more internal phosphate groups may be present. The pyrophosphates are discussed separately under anticalculus agents. The inorganic polyphosphate salts desired include tetrapolyphosphate and hexametaphosphate, among others. Polyphosphates larger than tetrapolyphosphate usually occur as amorphous glassy materials. Preferred in this invention are the linear "glassy" polyphosphates having the formula:

XO(XPO₃)ₙX

wherein X is sodium, potassium, or hydrogen and n averages from about 6 to about 125. It is further preferred that the particulate polyphosphate is sodium polyphosphate with an average chain length of from about 10 to about 30, preferably from about 15 to 25, more preferably from about 21 to about 23. Preferred are polyphosphates manufactured by FMC Corporation which are commercially known as Sodaphos (n≈6), Hexaphos (n≈13), and Glass H (n≈21). Hexaphos and Glass H are preferred with Glass H being the most preferred polyphosphate. These polyphosphates may be used alone or in an combination thereof. The phosphate sources are also described in more detail in Kirk & Othmer, *Encyclopedia of Chemical Technology,* Fourth Edition, Volume 18, Wiley-Interscience Publishers (1996).

The amount of polymeric surface agent required is an effective amount to provide the surface conditioning effects and to provide a "crunchy" texture. An effective amount of a polymeric surface active agent will typically be from 0.1% to 50%, preferably from 1% to 35%, more preferably from 2% to 25%, and most preferably from 5% to 15% by weight of the total chewing gum composition. In addition to creating the surface conditioning effects, the polymeric surface active agent has been found to associate with orally active metallic ions or species whilst maintaining their solubility and efficacy. For example, the polymeric surface active agent may form a complex with stannous fluoride or zinc and still provide the desired tartar control, stain control, and surface conditioning, in addition to not significantly prohibiting the efficacy of the stannous fluoride or zinc. The polymeric surface active agents may enhance the solubility of orally active metallic ions, such as zinc salts and stannous salts. The orally active metallic ions may provide several efficacious benefits to the chewing gum such as reduced gingivitis, plaque, and sensitivity and improved breath. In addition to not significantly reducing the efficacy of an orally active metallic ion, the polymeric surface active agents, particularly sodium polyphosphate, may reduce the amount of astringency created by the orally active metallic ion. This may be due to lower levels of mucosal protein precipitation in the mouth. This can be measured by in vivo testing and sensory scores. The preferred orally active metallic ions are zinc and tin or stannous. The orally active metallic ions are typically present in an amount of from about 0.01% to about 10%, preferably from about 0.05% to about 5%, more preferably from about 0.1% to about 1%, by weight of the chewing gum.

It may be desirable to have a sustained release of the polymeric surface active agent from the chewing gum. This may be accomplished by incorporating a cationic material whose polymeric surface active agent salt is less soluble in water than the sodium, potassium or hydrogen salts. By adding such cationic material, particularly divalent cationic materials such as calcium, the release rate of the polymeric surface active agent may be tailored to a required profile. The maximum level of cationic material incorporated is one cation per monomer unit forming the polymeric surface active agent. The level of cationic material incorporated is more preferably less than 0.5 cations per monomer unit forming the polymeric surface active agent. By weight percent, the polymeric surface active agent is generally present in at least about two times the cationic material, preferably about four times the cationic material, and more preferably at least about five times the cationic material. For example, the cationic material is typically present in an amount of up to about 10%, preferably from about 0.05% to about 5%, and more preferably from about 0.1% to about 3%, by weight of the chewing gum. The particulate polyphosphate material should have a minimum particle size such that they are retained by a 0.1mm mesh, preferably a 0.112mm mesh, more preferably a 0.16mm mesh, even more preferably a 0.18mm mesh and most preferably a 0.2mm mesh wherein the meshes are selected from the DIN 4188 mesh series . Furthermore the solid particulate materials should have a maximum particle size such that pass through a 2mm mesh, preferably a 1mm mesh, more preferably an 0.8mm mesh, even more preferably a 0.5mm mesh and most preferably a 0.4mm mesh, again wherein the meshes are selected from the DIN 4188 mesh series.

The solubility of the particulate polyphosphate should be at least 5g per 100ml at 25°C, preferably at least 8g, more preferably at least 10g, even preferably at least 15g per 100ml at 25°C. Thus the solid particulate should be "sparingly soluble", or preferably more soluble, wherein the term is defined as in the British Pharmacopoeia, 1999, Volume 1. Whilst there is no limit on the upper solubility of the polyphosphate it is preferred that it is not freely soluble in water otherwise it will dissolve too rapidly for a crunch to be experienced.

It is preferred that particulate polyphosphate materials have a hardness of greater than 1, preferably of 2 or greater, on the Mohs hardness scale. It is also preferred that the particulate polyphosphate materials for use in the second aspect of this invention are distributed evenly throughout the gum base. Additionally it is also preferred that, according to the second aspect of this invention, that the weight ratio of gum to particulate polyphosphate is in the range from 10:1 to 1:10, preferably from 5:1 to 1:5, more preferably from 5:1 to 1:1.

### Additional chewing gum components

In preparing the present chewing gum compositions, it is desirable to add one or more additional chewing gum components. Such materials are well known in the art and are readily chosen by one skilled in the art based on the physical and aesthetic properties desired for the chewing gum compositions being prepared. These carriers may be included at levels which do not interfere or prohibit the surface conditioning. The amount of polymeric surface active agent may be increased to account for the additional components. The additional chewing gum components typically comprise from 30% to 99%, preferably from 40% to 98%, and more preferably from 70% to 95%, by weight of the chewing gum composition.

Additional chewing gum components include carrier materials. The carrier materials are water insoluble materials which are typically not released in the mouth and water soluble materials which are released in the mouth. Water insoluble materials are typically used to form a chewing gum base.

An abrasive polishing material may be included in the chewing gum compositions. The abrasive polishing material contemplated for use in the compositions of the present invention can be any material which does not excessively abrade dentin. The abrasive polishing material should be formulated in the chewing gum composition so that it does not compromise the stability of any ingredients. Typical abrasive polishing materials include silica gels and precipitates; aluminas; water soluble phosphates (including orthophosphates, polymetaphosphates, and pyrophosphates); and mixtures thereof. Specific examples include dicalcium orthophosphate dihydrate, calcium pyrophosphate, tricalcium phosphate, calcium polymetaphosphate, insoluble sodium polymetaphosphate, hydrated alumina, beta calcium pyrophosphate, calcium carbonate, and resinous abrasive materials such as particulate condensation products of urea and formaldehyde. Mixtures of abrasives may also be used. The abrasive in the chewing gum compositions is generally from 1% to 70% and preferably from 5% to 50%, by weight of the chewing gum composition.

Another ingredient of the chewing gum composition is an elastomer or elastomer mixture. The elastomers useful in the present composition include styrene-butadiene rubber (SBR) and other elastomeric materials generally known in the art. Illustrative elastomers include SBR, synthetic gums or elastomers such as polyisobutylene and isobutylene-isoprene copolymers; natural gums or elastomers such as chicle, natural rubber, jelutong, balata, guttapercha, lechi caspi, sorva and mixtures thereof. The elastomer or elastomer mixture is generally present in an amount of from 2% to 30% and preferably from 5% to 25% by weight. When the total amount of elastomer is below 2% the base composition lacks elasticity, chewing texture, and cohesiveness whereas at amounts above about 30% the formulation is hard, rubbery and maintains a tight chew.

An optional but desirable ingredient of the chewing gum composition is a resin. The resin serves to plasticise the gum base. Suitable resins for use herein include polyvinyl acetate (PVA) and terpene resins, including polyterpene and polymers of alpha-pinene or beta-pinene, and mixtures thereof. The resin can conveniently be used at a level of from 3% to 25%, preferably from 5% to 20% by weight of the gum composition.

In addition to the resin component, the gum base compositions of the present invention preferably comprise a plasticiser in an amount up to 10%, preferably from 0.1% to 3% by weight of the gum composition. Suitable plasticisers include glyceryl triacetate, acetylated monoglyceride, glyceryl tributyrate, ethyl laurate, ethyl acetoacetate, diethyl tartrate, ethyl or butyl lactates, diethyl malate, ethyl oleate, castor oil, succinylated monoglycerides or mixtures thereof. Glyceryl triacetate and acetylated monoglyceride are preferred.

Various fats can also be included in the chewing gum compositions of the present invention. Preferred fats include the hydrogenated vegetable oils such as hydrogenated palm oil, hydrogenated soybean oil, hydrogenated cotton seed oil and various other hydrogenated vegetable oils and mixtures thereof. The fats can suitably be used at a level up to 20%, preferably from 1% to 10% by weight of the chewing gum composition.

A further desirable ingredient of the chewing gum base composition is an elastomer solvent. The elastomer solvent aids in softening the elastomer component. Such elastomer solvents include methyl, glycerol or pentaerythritol esters of rosins or modified rosins, such as hydrogenated, dimerized or polymerised rosins or mixtures thereof. Examples of elastomer solvents suitable for use herein include the pentaerythritol ester of partially hydrogenated wood rosin, pentaerythritol ester of wood rosin, glycerol ester of partially dimerized rosin, glycerol ester of polymerised rosin, glycerol ester of tall oil, wood or gum rosin, glycerol ester of partially hydrogenated rosin, methyl ester of partially hydrogenated rosin, and mixtures thereof. The elastomer solvent can be employed in an amount ranging from 2% to 50%, preferably from 10% to 35% by weight of the chewing gum.

The gum base compositions can also include one or more waxes. Suitable waxes include paraffin wax; microcrystalline wax; Fischer-Tropsch paraffin; natural waxes such as candellilla, camauba and beeswax; polyolefin waxes such as polyethylene wax; and mixtures thereof. The waxes can be present in levels up to 25%, preferably from 5% to 20% by weight of the gum composition.

The chewing gum also preferably includes an emulsifier. Suitable emulsifiers include glycerol monostearate, lecithin, fatty acid monoglycerides, diglycerides, propylene glycol monostearate and mixtures thereof. The emulsifier is employed in amounts up to 10% and preferably from 2% to 6% by weight of the chewing gum.

A variety of softeners can also be employed in the chewing gum compositions of the present invention. Suitable softeners include fatty materials such as lanolin, stearic acid, sodium stearate and potassium stearate; polyhydric alcohols such as glycerine, propylene glycol, and the like; and mixtures thereof. The softeners can suitably be used at a total level of up to 30%, preferably from 0.1% to 10% by weight of the chewing gum. In a preferred embodiment, the chewing gum composition comprises a fatty softener selected from stearic acid, sodium stearate, potassium stearate and mixtures thereof in an amount of from 0.1% to 10% by weight of the chewing gum. Preferably, the fatty softener is stearic acid. The gum base composition may further comprise a polyhydric alcohol. If present, the polyhydric alcohol is present in an amount of from 0.5% to 25%, more preferably from 1% to 10% by weight of the chewing gum. Such materials, when incorporated into the gum base, assist in modifying the texture and consistency properties. In particular, they help to soften the chew and to maintain chew softness over an extended period of time.

Bulking agents, such as fillers, can also be employed in the chewing gum. Suitable fillers and bulking agents are generally non-abrasive, preferably with an average particle size less than 5 µm, more preferably less than 3 µm and especially less than 1 µm. Illustrative bulking agents include calcium carbonate or ground limestone, talc, aluminium hydroxide, alumina, aluminium silicates, dicalcium phosphate and mixtures thereof. Where present, the filler can be used in levels up to 50%, preferably up to 30%, most preferably from up to 10% by weight of the chewing gum.

Suitable bulk sweeteners are monosaccharides, disaccharides, and polysaccharides such as xylose, ribose, glucose, mannose, galactose, fructose, dextrose, sucrose, sugar maltose, fructo oligo saccharide syrups, partially hydrolysed starch, or corn syrup solids. Preferred sweetening agents are sugar alcohols such as sorbitol, xylitol, mannitol, maltitol, isomalt, hydrogenated starch hydrolisate, inulin, and other non-carigenic edible polyols such as glycerin and erythritol and mixtures thereof.

In general, the amount of sweetener will vary with the sweetener used and desired amount of sweetener selected for a particular chewing gum. This amount will normally vary from 0.01% when using a high intensity sweetener to 80% by weight of the chewing gum composition when using an easily extractable bulk sweetener. The bulk sweeteners described above, are preferably used in amounts of 10% to 80% by weight and most preferably about 30% to about 70% by weight. These amounts are ordinarily necessary to achieve a desired level of sweetness independent from the flavor level achieved from the flavoring agents.

In preferred embodiments, the chewing gum composition further comprises a high intensity sweetener. Suitable high intensity sweeteners include: dipeptide based sweeteners such as L-aspartyl-L-phenylalanine methyl ester (Aspartame), and equivalents described in U.S. Pat. No. 3,492,131, L-α-aspartyl-N-(2,2,4,4-tetramethyl-3-thietanyl)-D-alaninamide hydrate (Alitame) and the like; the soluble saccharin salts, i.e., sodium or calcium saccharin salts; cyclamate salts, acesulfame-K and the like; the free acid form of saccharin; chlorinated derivatives of sucrose such as chlorodeoxysucrose and the like; and protein based sweeteners, such as Thaumatin (talin). The high intensity sweeteners described can be added in amounts of from 0.01% to 2.0% and most preferably from 0.05% to 0.5% by weight of the chewing gum composition. Using a high intensity sweetener within the gum base may prolong the flavor of the finished gum composition during chewing.

The chewing gum can also include colorants and pigments, such as titanium dioxide. In general, the gum can contain up to about 2% of pigment and / or colorant. Anti-oxidants can also be included in the gum, at a level of up to about 0.5%. Suitable anti-oxidants are butylated hydroxyanisole, butylated hydroxytoluene, propyl gallate, ascorbic acid and tocopherols.

Flavoring agents well known in the chewing gum art can be added to the chewing gum compositions of the invention. These flavoring agents can be chosen from synthetic flavoring liquid and/or oils derived from plants leaves, flowers, fruits and so forth, and combinations thereof. Representative flavoring liquids include: spearmint oil, cinnamon oil, oil of wintergreen (methylsalicylate) and peppermint oils. Also useful are artificial, natural or synthetic fruit flavors such as citrus oil including lemon, orange, banana, grape, lime, apricot and grapefruit and fruit essences including apple, strawberry, cherry, orange, pineapple and so forth; bean and nut derived flavors such as coffee, cocoa, cola, peanut, almond and so forth. Additionally, flavor adsorbed onto a hdrophillic matrix may be included e.g. "spray-dried" flavors. Furthermore encapsulated flavors may be included. Also included in the term flavorant are sensates and coolants. Preferred coolants include MGA, Physcool®, WS-3, WS-23, TK-10, and combinations thereof.

The amount of flavorant employed is normally a matter of preference subject to such factors as flavor type, base type and strength desired. In general, amounts up to about 4% by weight and preferably 0.05% to 3.0% by weight of the chewing gum composition are usable with amounts of 0.8% to 2.5% being preferred.

Water employed in the preparation of commercially suitable chewing gum compositions should preferably be of low ion content and free of organic impurities. Water will generally comprise less than 10%, preferably from 0.01% to 5%, and more preferably from 0.1% to 3%, by weight of the composition herein. The amounts of water include the free water which is added plus that which is introduced with other materials, such as with sorbitol, silica, and solutions.

The chewing gums of the present invention may also include other agents, such as antimicrobial agents. The chewing gum composition may include an orally active metallic ion as an antimicrobial agent, particularly salts of zinc, tin and silver and copper.

Other antimicrobial agents include the water insoluble non-cationic antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides. The water soluble antimicrobials include quaternary ammonium salts and bis-biquanide salts, among others. Triclosan monophosphate is an additional water soluble antimicrobial agent. The quaternary ammonium agents include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethylstearyl ammonium chloride, cetyl pyridinium chloride, cetyl pyridinium saccharinate, quatemized 5-amino-1,3-bis(2-ethyl-hexyl)-5-methyl hexa hydropyrimidine, benzalkonium chloride, benzethonium chloride and methyl benzethonium chloride are exemplary of typical quaternary ammonium antibacterial agents. Other compounds are bis[4-(R-amino)-1-pyridinium] alkanes as disclosed in U.S. Patent 4,206,215, issued June 3, 1980, to Bailey. Other antimicrobials such as copper bisglycinate, copper glysinate, zinc citrate, zinc citrate-maleate, zinc lactate, hexetidine, hexamadine, furanones, and phalimido-peroxycaproic acid may also be included. Also useful are enzymes, including endoglycosidase, papain, dextranase, mutanase, and mixtures thereof. Such agents are disclosed in U.S. Patent 2,946,725, Jul. 26, 1960, to Norris et al. and in U.S. Patent 4,051,234, September. 27, 1977 to Gieske et al.,. Specific antimicrobial agents include chlorhexidine, triclosan and its derivatives including triclosan monophosphate, triclosan diphosphate, and phenolated triclosan and flavor oils such as thymol, geraniol, eugenol, and biosol. Triclosan and other agents of this type are disclosed in Parran, Jr. et al., U.S. Patent 5,015,466, issued May 14, 1991, and U.S. Patent 4,894,220, Jan. 16, 1990 to Nabi et al.,. These agents may be present at levels of from 0.01% to 10%, preferably from 0.05% to 5%, and more preferably from 0.1% to 2%, by weight of the chewing gum composition.

Optional agents that may be used in combination with the polyphosphate include materials known to be effective in reducing calcium phosphate mineral deposition related to calculus formation. Pyrophosphate salts may be used in the present invention as anticalculus agents or as buffering agents, as long of the surface conditioning effects of the polymeric surface active agent is not eliminated. The amount of pyrophosphate salt useful in making these compositions is any tartar control effective amount, and is generally from about 1.5% to about 15%, preferably from about 2% to about 10%, and most preferably from about 2.5% to about 8%, by weight of the chewing gum composition. Other agents included are synthetic anionic polymers [including polyacrylates and copolymers of maleic anhydride or acid and methyl vinyl ether (e.g., Gantrez), as described, for example, in U.S. Patent 4,627,977, to Gaffar et al., as well as, e.g., polyamino propoane sulfonic acid (AMPS)], zinc citrate trihydrate, diphosphonates (e.g., EHDP; AHP), polypeptides (such as polyaspartic and polyglutamic acids), and mixtures thereof.

The present invention may also include buffering agents to adjust the pH of the chewing gum and may help to stabilize the polymeric surface active agent. Other potential ingredients include a fluoride ion source. An alkali metal bicarbonate salt, surfactants, whitening agents such as peroxide or percarbonate, coloring agents, xylitol, thickening materials, binders, humectants, absorbents such as activated carbon, silica absorbents, cyclodextrins, and zeolites and combinations thereof, may also be included in the chewing gum composition.

The chewing gum composition may be in the form of a chiclet or other form that contains a outer coating or shell around the central portion or gum base of the chewing gum. The outer coating may be hard or crunchy. Typically, the outer coating will comprise sorbitol, maltitol, xylitol, isomalt, and other crystallisable polyols. The outer coating may also contain small amounts of water and gum arabic. A polyol coating can be further coated with wax. The present invention may have the polymeric surface active agent present in the chewing gum base, the outer coating, or both.

It may be desirable to have a rapid release of the polymeric surface active agent from the chewing gum. This may be accomplished by incorporating some or all of the polymeric surface active agent into the outer coating. To further increase the rate of release of the polymeric surface active agent from the outer coating, the particles are processed in a manner to result in a microporous sized porosity.

### Examples & Method of Manufacturing

The following examples further describe and demonstrate embodiments within the scope of the present invention. These examples are given solely for the purpose of illustration and are not to be construed as limitations of the present invention.

| Material Name (INCI) | A | B | C | | D | |
|---|---|---|---|---|---|---|
| | | | Core | Coating | Core | Coating |
| Sorbitol | 25.850 | 18.350 | 33.350 | | 33.350 | |
| Xylitol | 16.700 | 16.700 | 16.700 | | 16.700 | |
| Gum base (e.g. Prestige-PL, Cafosa) | 28.000 | 28.000 | 28.000 | | 28.000 | |
| Sodium polyphosphate, n=21; Glass H (FMC) | 7.500 | 15.000 | 0.000 | 37.500 | 0.000 | |
| Glass H (microporous sized) | | | | | | 37.500 |
| Hydrogenated starch hydrolisate (85% solids) | 8.000 | 8.000 | 8.000 | | 8.000 | |
| Glycerin | 7.000 | 7.000 | 7.000 | | 7.000 | |
| Mannitol | 5.000 | 5.000 | 5.000 | | 5.000 | |
| Maltitol | | | | 62.250 | | 62.250 |
| Ethyl cellulose (Ethocell, Dow Corning) | | | | | | |
| Zinc lactate dihydrate | | | | | | |
| Sodium polyphosphate, n=6; Sodaphos (FMC) | | | | | | |
| Sodium polyphosphate, n=13; Hexaphos (FMC) | | | | | | |
| Poloxamer 407 | | | | | | |
| Flavor | 1.600 | 1.600 | 1.600 | 0.250 | 1.600 | 0.250 |
| Aspartame | 0.200 | 0.200 | 0.200 | | 0.200 | |
| Spray dried flavor | 0.150 | 0.150 | 0.150 | | 0.150 | |
| **TOTAL** | **100.000** | **100.000** | **100.000** | **100.000** | **100.000** | **100.000** |
| | | | | | | |
| **% OF TOTAL COMPOSITION** | **100.000** | **100.000** | **80.000** | **20.000** | **80.000** | **20.000** |

| Material Name (INCI) | E | F | G | H | I | J | K |
|---|---|---|---|---|---|---|---|
| Sorbitol | 15.850 | 25.123 | 18.350 | 23.350 | 23.850 | 23.850 | 24.719 |
| Xylitol | 16.700 | 16.700 | 16.700 | 16.700 | 16.700 | 16.700 | 16.700 |
| Gum base (e.g. Prestige-PL, Cafosa) | 28.000 | 28.000 | 28.000 | 28.000 | 28.000 | 28.000 | 28.000 |
| Sodium polyphosphate, n=21; Glass H (FMC) | 7.500 | 7.500 | | | 7.500 | 7.500 | 7.500 |
| Hydrogenated starch hydrolisate (85% solids) | 8.000 | 8.000 | 8.000 | 8.000 | 8.000 | | 8.000 |
| Glycerin | 7.000 | 7.000 | 7.000 | 7.000 | 7.000 | 7.000 | 7.000 |
| Mannitol | 5.000 | 5.000 | 5.000 | 5.000 | 5.000 | 5.000 | 5.000 |
| Maltitol | | | | | | 8.000 | |
| Ethyl cellulose (Ethocell, Dow Corning) | 10.000 | | | | | | |
| Zinc lactate dihydrate | | 0.727 | | | | | |
| Sodium polyphosphate, n=6; Sodaphos (FMC) | | | 15.000 | | | | |
| Sodium polyphosphate, n=13; Hexaphos (FMC) | | | | 10.000 | | | |
| Poloxamer 407 | | | | | 2.000 | 2.000 | |
| Flavor | 1.600 | 1.600 | 1.600 | 1.600 | 1.600 | 1.600 | 1.600 |
| Aspartame | 0.200 | 0.200 | 0.200 | 0.200 | 0.200 | 0.200 | 0.200 |
| Spray dried flavor | 0.150 | 0.150 | 0.150 | 0.150 | 0.150 | 0.150 | 0.150 |
| Calcium chloride | | | | | | | 0.754 |
| | | | | | | | |
| **TOTAL** | **100.000** | **100.00** | **100.00** | **100.00 0** | **100.00 0** | **100.00 0** | **100.00 0** |
| | | | | | | | |
| **% OF TOTAL COMPOSITION** | **100.000** | **100.00** | **100.00** | **100.000** | **100.000** | **100.000** | **100.000** |

### Making Instructions

### Products A, B

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol, Glass-H and aspartame and mix for 3 minutes. Add flavor and mix for 3 minutes.

### Products C, D

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol aspartame and mix for 3 minutes. Add flavor and mix for 3 minutes. Store for ∼1 week at 15-20°C, 30-60% RH (conditioning). Tumble chewing gums in coater-drier with maltitol solution and Glass-H. Maintain tumbling until surface is dry.

### Product E

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol, Glass-H in encapsulated ethyl celullose and aspartame and mix for 3 minutes. Add flavor and mix for 3 minutes.

### Product F

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol, Glass-H, zinc lactate, and aspartame and mix for 3 minutes. Add flavor and mix for 3 minutes.

### Product G, H

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol, sodaphos, hexaphos, and aspartame and mix for 3 minutes. Add flavor and mix for 3 minutes.

### Product I

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol and mix for 3 minutes. Add second 50% of xylitol, Glass-H, Poloxamer 407, and aspartame and mix for 3 minutes. Add flavor and mix for 3 minutes.

### Product J

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 7 minutes. Add 50% sorbitol and mix for 3 minutes. Add second 50% of xylitol, Glass-H and aspartame and mix for 3 minutes. Add flavor and mix for 3 minutes.

### Product K

Heat gum base to ∼45°C to soften. Maintain mixer vessel cavity at ∼45°C during entire mixing process. Add gum base to mixing cavity of double sigma blade mixer and mix for 5 minutes. Add mannitol and spray-dried menthol. Mix for 2 minutes. Add glycerin and mix for 2 minutes. Add 50% of xylitol and mix for 2 minutes. Add hydrogenated starch hydrolisate and mix for 5 minutes. Add 50% sorbitol, calcium chloride and mix for 3 minutes. Add second 50% of xylitol, Glass-H and aspartame and mix for 3 minutes. Add flavor and mix for 3 minutes.

## Claims

1. A chewing gum comprising greater than 10% gum base, comprising one or more elastomers, resins or waxes and mixtures thereof, and from 0.1% to 50% of a linear polyphosphate having a chain length of four or more phosphate groups, **characterised in that** the polyphosphate is a particulate solid and has:
a) a particle size such that it passes through a 2mm mesh and is retained by a 0.1mm mesh; and
b) an aqueous solubility of at least 5g per 100ml at 25°C.

2. A chewing gum according to Claim 1 which comprises from 0.5% to 30%, preferably from 1% to 15%, more preferably from 5% to 12%, by weight, of the particulate polyphosphate.

3. A chewing gum according to Claim 1 or Claim 2 wherein the particulate polyphosphate has a particle size such that it passes through a 1mm mesh, preferably a 0.8mm mesh, more preferably 0.5mm mesh and even more preferably a 0.4mm mesh.

4. A chewing gum according to any of Claims 1 to 3 wherein the particulate polyphosphate has a particle size such that it is retained by a 0.112mm mesh, preferably a 0.16mm mesh, more preferably a 0.18mm mesh and even more preferably a 0.2mm mesh.

5. A chewing gum according to any of Claims 1 to 4 wherein the particulate polyphosphate is sodium polyphosphate with an average chain length of from 10 to 30, preferably from 15 to 25, more preferably from 21 to 23.

6. A chewing gum according to any of Claims 1 to 5 wherein the particulate polyphosphate has an aqueous solubility of at least 8g, preferably at least 10g, more preferably at least 15g per 100ml at 25°C.

7. A chewing gum according to any of Claims 1 to 6 wherein the particulate polyphosphate has a hardness of greater than 1, preferably 2, or greater, when measured using the Mohs hardness scale.

8. A chewing gum according to any of Claims 1 to 7 wherein the particulate polyphosphate is dispersed throughout the chewing gum composition.

9. A chewing gum according to any of Claims 1 to 8 wherein the weight ratio of gum to particulate polyphosphate is in the range from 10:1 to 1:10, preferably from 5:1 to 1:5, more preferably from 5:1 to 1:1.

10. A chewing gum according to any of Claims 1 to 9 comprising a hard outer coating of sorbitol, maltitol, xylitol, isomalt, or other crystallisable polyols.

## Patentansprüche

1. Kaugummi, umfassend mehr als 10% Gummibasis, umfassend ein oder mehrere Elastomere, Harze oder Wachse und Mischungen hiervon, und 0,1% bis 50% eines linearen Polyphosphats mit einer Kettenlänge von 4 oder mehr Phosphatgruppen, **dadurch gekennzeichnet, dass** das Polyphosphat ein teilchenförmiger Feststoff ist und
a) eine Teilchengröße besitzt, so dass es eine 2 mm Sieböffnung passiert und von einer 0,1 mm Sieböffnung zurückgehalten wird; und
b) eine Wasserlöslichkeit von mindestens 5 g pro 100 ml bei 25°C besitzt.

2. Kaugummi nach Anspruch 1; umfassend 0,5% bis 30%, vorzugsweise 1% bis 15%, weiter vorzugsweise 5% bis 12%, bezogen auf Gewicht, des teilchenförmigen Polyphosphats.

3. Kaugummi nach Anspruch 1 oder Anspruch 2, wobei das teilchenförmige Polyphosphat eine solche Teilchengröße besitzt, dass es durch eine 1 mm Sieböffnung, vorzugsweise eine 0,8 mm Sieböffnung, weiter vorzugsweise 0,5 mm Sieböffnung, und noch weiter vorzugsweise ein 0,4 mm Sieböffnung, passiert.

4. Kaugummi nach mindestens einem der Ansprüche 1 bis 3, wobei das teilchenförmige Polyphosphat eine solche Teilchengröße besitzt, dass es durch eine 0,112 mm Sieböffnung, vorzugsweise eine 0,16 mm Sieböffnung, weiter vorzugsweise eine 0,18 mm Sieböffnung, und noch weiter vorzugsweise eine 0,2 mm Sieböffnung, zurückgehalten wird.

5. Kaugummi nach mindestens einem der Ansprüche 1 bis 4, wobei das teilchenförmige Polyphosphat Natriumpolyphosphat mit einer durchschnittlichen Kettenlänge von 10 bis 30, vorzugsweise 15 bis 25, weiter vorzugsweise 21 bis 23, ist.

6. Kaugummi nach mindestens einem der Ansprüche 1 bis 5, wobei das teilchenförmige Polyphosphat eine Wasserlöslichkeit von mindestens 8 g, vorzugsweise mindestens 10 g, weiter vorzugsweise mindestens 15 g pro 100 ml bei 25°C aufweist.

7. Kaugummi nach mindestens einem der Ansprüche 1 bis 6, wobei das teilchenförmige Polyphosphat eine Härte von größer als 1, vorzugsweise 2, oder größer besitzt, gemessen unter Anwendung der Mohs-Härteskala.

8. Kaugummi nach mindestens einem der Ansprüche 1 bis 7, wobei das teilchenförmige Polyphosphat in der Kaugummizusammensetzung dispergiert ist.

9. Kaugummi nach mindestens einem der Ansprüche 1 bis 8, wobei das Gewichtsverhältnis von Gummi zu teilchenförmigem Polyphosphat im Bereich von 10:1 bis 1:10, vorzugsweise 5:1 bis 1:5, weiter vorzugsweise 5:1 bis 1:1, liegt.

10. Kaugummi nach mindestens einem der Ansprüche 1 bis 9, umfassend eine harte Außenbeschichtung aus Sorbit, Maltit, Xylit, Isomalt oder anderen kristallisierbaren Polyolen umfasst.

## Revendications

1. Gomme à mâcher comprenant plus de 10% d'une base de gomme, comprenant un ou plusieurs élastomères, résines ou cires, et leurs mélanges, et de 0,1% à 50% d'un polyphosphate linéaire ayant une longueur de chaîne d'au moins quatre groupes phosphate, **caractérisée en ce que** le polyphosphate est un solide particulaire et possède :
a) une granulométrie telle qu'il traverse un tamis d'ouverture de maille 2 mm et qu'il est refusé sur un tamis d'ouverture de maille 0,1 mm; et
b) une solubilité en milieu aqueux d'au moins 5 g dans 100 ml à 25°C.

2. Gomme à mâcher selon la revendication 1 qui comprend de 0,5% à 30%, de préférence de 1% à 15%, plus préférablement de 5% à 12%, en poids, du polyphosphate particulaire.

3. Gomme à mâcher selon la revendication 1 ou la revendication 2 dans laquelle le polyphosphate particulaire possède une granulométrie telle qu'il traverse un tamis d'ouverture de maille 1 mm, de préférence un tamis d'ouverture de maille 0,8 mm, plus préférablement un tamis d'ouverture de maille 0,5 mm et encore mieux un tamis d'ouverture de maille 0,4 mm.

4. Gomme à mâcher selon l'une quelconque des revendications 1 à 3 dans laquelle le polyphosphate particulaire possède une granulométrie telle qu'il est refusé sur un tamis d'ouverture de maille 0,112 mm, de préférence un tamis d'ouverture de maille 0,16 mm, plus préférablement un tamis d'ouverture de maille 0,18 mm et plus préférablement un tamis d'ouverture de maille 0,2 mm.

5. Gomme à mâcher selon l'une quelconque des revendications 1 à 4 dans laquelle le polyphosphate particulaire est un polyphosphate de sodium ayant une longueur moyenne de chaîne de 10 à 30, de préférence de 15 à 25, plus préférablement de 21 à 23.

6. Gomme à mâcher selon l'une quelconque des revendications 1 à 5 dans laquelle le polyphosphate particulaire a une solubilité en milieu aqueux d'au moins 8 g, de préférence d'au moins 10 g, plus préférablement d'au moins 15 g, pour 100 ml à 25°C.

7. Gomme à mâcher selon l'une quelconque des revendications 1 à 6 dans laquelle le polyphosphate particulaire a une dureté supérieure à 1, de préférence de 2 ou plus, mesurée sur l'échelle de dureté Mohs.

8. Gomme à mâcher selon l'une quelconque des revendications 1 à 7 dans laquelle le polyphosphate particulaire est dispersé dans la composition de gomme à mâcher.

9. Gomme à mâcher selon l'une quelconque des revendications 1 à 8 dans laquelle le rapport pondéral de la gomme au polyphosphate particulaire est dans la gamme de 10:1 à 1:10, de préférence de 5:1 à 1:5, plus préférablement de 5:1 à 1:1.

10. Gomme à mâcher selon l'une quelconque des revendications 1 à 9 comprenant un enrobage externe dur de sorbitol, de maltitol, de xylitol, d'isomalt ou d'autres polyols cristallisables.
